# EUROPEAN PATENT SPECIFICATION

(11) **EP 2 887 899 B1**
(45) Date of publication and mention of the grant of the patent: **02.08.2017**
(21) Application number: 13833165.7
(22) Date of filing: 27.08.2013
(51) Int. Cl.: A61B 17/86, A61B 17/70, A61F 2/44

(54) **IMPROVED FENESTRATED BONE SCREW**
VERBESSERTE FENESTRIERTE KNOCHENSCHRAUBE
VIS À OS FENESTRÉE PERFECTIONNÉE

(30) Priority: 27.08.2012 US 201213595181
(43) Date of publication of application: 01.07.2015
(73) Proprietor: Globus Medical, Inc., Audubon, PA 19403 (US)
(72) Inventor: BLACK, Michael, Phoenixville, PA 19460 (US)
(74) Representative: Morabito, Sara
(86) International application number: PCT/US2013/056807
(87) International publication number: WO 2014/035973

(56) References cited:
- WO-A2-02/085181
- WO-A2-2010/099239
- US-A1- 2001 021 852
- US-A1- 2004 015 172
- US-A1- 2005 203 530
- US-A1- 2006 122 604
- US-A1- 2006 122 604
- US-A1- 2006 155 286
- US-A1- 2011 213 426
- US-A1- 2012 029 578
- US-A1- 2012 197 311

## Description

### FIELD OF THE INVENTION

The present invention is directed to bone screws as defined in the claims.

### BACKGROUND OF THE INVENTION

Numerous procedures exist to alleviate pain caused by bone disease, trauma and fracture. During surgery, a number of implants, such as plates and stabilization systems, are used during treatment. These systems rely on bone screws that secure the implants to bone. There is thus a need to provide improved bone screws.

Document WO02085181 A2 discloses a hollow bone screw comprising a slot with two angled sections.

### SUMMARY OF THE INVENTION

Various embodiments of orthopedic implants are provided. In some embodiments, a bone screw is provided comprising a head portion comprising one or more slits. The bone screw further comprises a shaft portion connected to the head portion, the shaft portion comprising a plurality of threads and a tapered distal end. The shaft portion further comprises a fenestration having a first section and a second section, wherein the first section extends along a first direction and the second section extends along a second direction.

In other embodiments, a bone screw is provided comprising a head portion comprising one or more slits. The bone screw further comprises a shaft portion connected to the head portion, wherein the shaft portion comprises a plurality of threads. The shaft portion further comprises a curved fenestration having at least one arc pattern along a length of the shaft portion.

In other embodiments, a bone screw is provided comprising a head portion. The bone screw further comprises a shaft portion connected to the head portion, wherein the shaft portion comprises a plurality of threads. The shaft portion further comprises a fenestration having a length that extends along a majority of the length of the bone screw.
In a further example not falling within the scope of the claims it is provided a bone screw comprising: a head portion comprising one or more slits; and a shaft portion connected to the head portion, the shaft portion comprising a plurality of threads, the shaft portion further comprising a curved fenestration having at least one arc pattern along a length of the shaft portion.
In a version the curved fenestration includes at least two arc patterns formed along a length of the shaft portion.
In a further version of the bone screw, the shaft portion comprises dual-diameter threading.
In a further aspect of the invention it is provided a bone screw comprising: a head portion; and a shaft portion connected to the head portion, the shaft portion comprising a plurality of threads, the shaft portion further comprising a fenestration having a length that extends along a majority of the length of the bone screw.

### BRIEF DESCRIPTION OF THE DRAWINGS

The invention will be more readily understood with reference to the embodiments thereof illustrated in the attached figures, in which only the embodiments of figures 1A-1C fall within the scope of the claims:
FIGS. 1A-1C illustrate an improved fenestrated screw having an angled fenestration according to some embodiments.
FIGS. 2A-2C illustrate an improved fenestrated screw having a curved fenestration and not falling within the scope of the claims.
FIGS. 3A-3C illustrate an improved fenestrated screw having a straight, continuous fenestration and not falling within the scope of the claims.
FIGS. 4A-4C illustrate an improved fenestrated screw having parallel fenestrations and not falling within the scope of the claims.

### DETAILED DESCRIPTION OF THE ILLUSTRATED EMBODIMENTS

Embodiments of the invention will now be described. The following detailed description of the invention is not intended to be illustrative of all embodiments. In describing embodiments of the present invention, specific terminology is employed for the sake of clarity. However, the invention is not intended to be limited to the specific terminology so selected. It is to be understood that each specific element includes all technical equivalents that operate in a similar manner to accomplish a similar purpose.

The present application describes bone screws having improved mechanical properties. In particular, the bone screws described herein have unique fenestrations that can accommodate and guide different material (e.g., bone cement) through different portions of the bone screw, thereby increasing the effectiveness of the material.

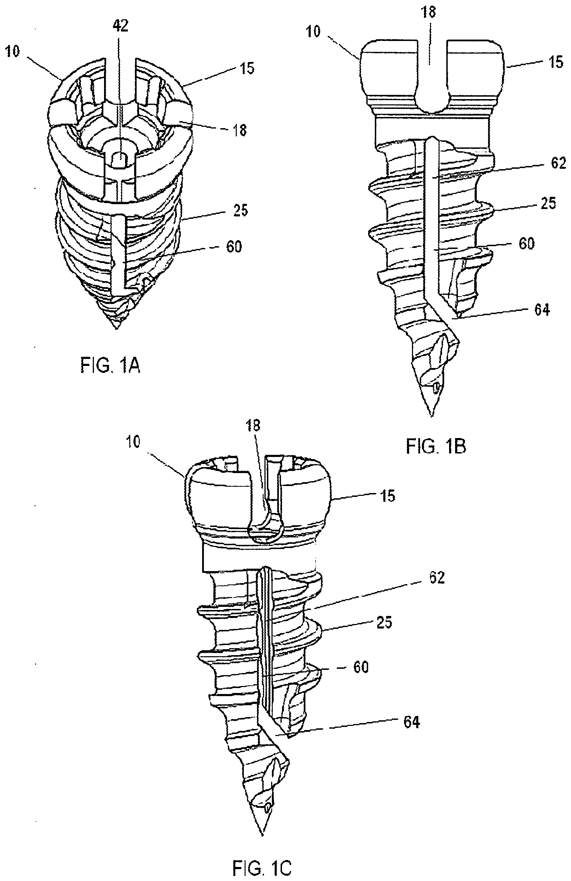

FIGS. 1A-1C illustrate an improved fenestrated screw having an angled fenestration according to some embodiments. The fenestrated screw 10 includes a head portion 15 connected to a threaded shaft portion 25. The threaded shaft portion 25 includes an angled fenestration 60 that extends along a longitudinal axis of the fenestrated screw 10, as discussed in more detail below.

The fenestrated screw 10 includes a head portion 15 having one or more openings or slits 18 formed therein. In some embodiments, the slits 18 are configured to allow for compressibility of the head portion 15. In addition, in other embodiments, the slits 18 are configured to accommodate one or more instruments, such as an insertion tool, for securely gripping the head portion 15 of the screw. As shown in FIG. 1A, the slits 18 are positioned symmetrically around the head portion 15 of the screw 10. However, in other embodiments, the slits 18 need not be positioned symmetrically around the head of the screw. As shown in FIG. 1A, the screw 10 includes four slits 18. However, in other embodiments, the screw can contain two, three, five or more slits.

The head portion 15 is operably connected to a threaded shaft portion 25. In some embodiments, the head portion 15 is formed monolithically with the shaft portion 25. In other embodiments, the head portion 15 is formed separately and then joined with the shaft portion 25. The shaft portion 25 is configured to have a tapered distal end that can be inserted into a vertebral body. In some embodiments, the threaded shaft portion 25 comprises a single thread, while in other embodiments, the threaded shaft portion 25 comprises a multi-diameter (e.g., dual-diameter) thread.

As shown in the top perspective view of FIG. 1A, the fenestrated screw 10 is cannulated such that an inner opening 42 extends from the head portion 15 to the shaft portion 25 of the screw 10. In some embodiments, the inner opening 42 can be open and is in fluid contact with the fenestration 60 (discussed in more detail below). Accordingly, material that is deposited through the inner opening 42, such as bone cement, can be advantageously distributed through the fenestration 60.

The fenestrated screw 10 includes a novel, angled fenestration 60. As shown in FIG. 1B, the fenestration 60 can extend along a longitudinal axis of the bone screw 10. In some embodiments, the fenestration 60 can extend along a majority of the length of the threaded shaft 25, thereby advantageously accommodating material along a majority of the length of the bone screw. For example, injected bone cement material, which can be used to secure the fenestrated screw 10 in bone, can now extend along a majority of the length of the bone screw 10, thereby improving the securing of the fenestrated screw in bone. In some embodiments, the fenestration 60 opens to the cannulated mid-portion of the fenestrated screw, such that material that is deposited through the cannulated portion can advantageously seep into the fenestration 60, and vice versa.

As shown best in FIGS. 1B and 1C, the fenestration 60 of the fenestrated screw 10 includes a first section 62 that extends along one direction and a second section 64 that extends along a different direction. In the illustrated embodiment, the first section 62 of the fenestration transitions into the second section 64 via an angled corner. As shown in the illustrated embodiments, the first section 62 comprises a straight portion that extends along a longitudinal length of the threaded shaft 25, which then transitions via an angled corner into the angled second section 64. In some embodiments, the angled corner of the fenestration 60 advantageously serves as a guide to direct material, such as fluids, along a certain direction along the length of the fenestration. In some embodiments, the first section 62 of the fenestration 60 can transition into the second section 64 via a sharp angle.

As shown in the side view of the screw 10 in FIG. 1B, the angled fenestration 60 can be formed such that the first section 62 of the fenestration 60 is formed in an interior portion of the screw 10 (e.g., along the middle longitudinal axis of the screw), while the second section 64 is formed to extend from an interior portion of the screw 10 to an outer edge of the screw 10. In some embodiments, the fenestration 60 effectively splits a portion of the threaded shaft 25 into two bodies. In other embodiments not shown, the fenestrated screw 10 can have an angled fenestration 60 that is formed completely in an interior portion of the screw 10 without extending to an outer edge of the screw 10. As the fenestration 60 can be made large and angled, this advantageously reduces the possibility of axial rotation and/or general screw toggling once cement or other biomaterial dries or fuses along the fenestration.

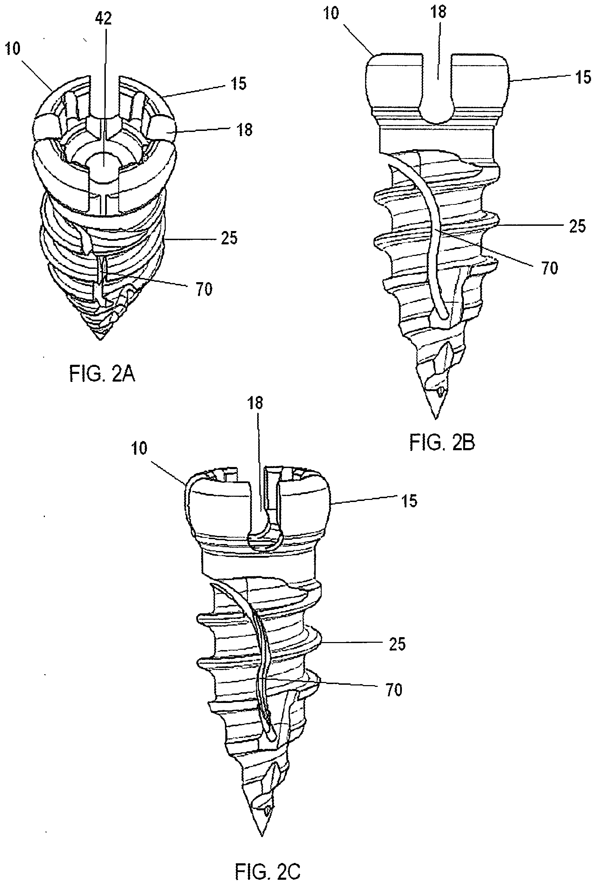

FIGS. 2A-2C illustrate an improved fenestrated screw having a curved fenestration not falling within the scope of the claims. Like the fenestrated screw discussed in FIGS. 1A-1C, the fenestrated screw 10 in FIGS. 2A-2C comprises a head portion 15, a shaft portion 25 and a fenestration therethrough. The fenestration 70, however, does not have an edge or corner, but rather is comprised of a continuous curve.

As shown in FIG. 2B and 2C, the fenestration 70 comprises a continuously curved opening or window formed along a portion of the shaft portion 25. In some embodiments, the fenestration 70 extends along a majority of the length of the shaft portion 25, thereby allowing material to effectively seep along a majority of the length of the screw 10. As shown in FIG. 2B, the fenestration 70 advantageously extends from an interior portion of the screw 10 all the way to an outer edge of an exterior portion of the screw 10. In addition, as shown in the illustrated embodiments, the fenestration 70 comprises at least two arcuate portions. Such arcuate portions can advantageously provide a good distribution of material through the fenestrations, while properly maintaining sufficient strength in the fenestrated screw 10. However, one skilled in the art will appreciate that it is possible to have a fenestration 70 with a single arc or more than two arcs. Furthermore, in some embodiments, the fenestration 70 opens to and is in fluidic contact with the inner opening 42 of the screw 10. As the fenestration 70 can be made large and curved, this advantageously reduces the possibility of axial rotation and/or general screw toggling once cement or other biomaterial dries or fuses along the fenestration.

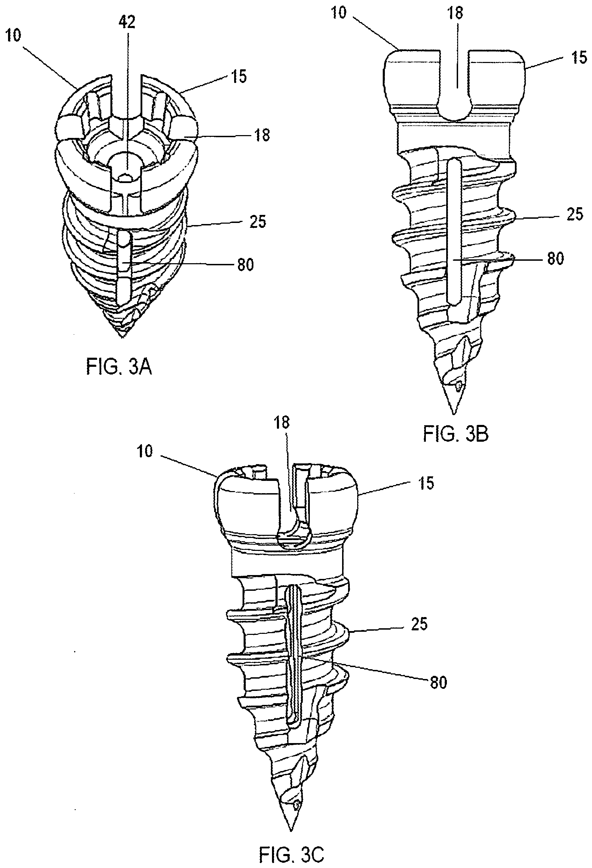

FIGS. 3A-3C illustrate an improved fenestrated screw having a straight, continuous fenestration not falling within the scope of the claims. Like the screws discussed above, fenestrated screw 10 includes a head 15 and a shaft 25. The screw 10 also includes a fenestration 80. However, the fenestration 80 comprises a substantially straight window or opening that is formed along a longitudinal length of the screw 10. While the fenestration 80 is substantially vertical and aligned along the mid-line of the screw, in other embodiments, the fenestration can be non-vertical (e.g., diagonal) and/or can be shifted away from the mid-line of the screw. In some embodiments, the fenestration 80 extends along a majority of the length of the shaft 25. Furthermore, in some embodiments, the fenestration 80 opens to and is in fluidic contact with the inner opening 42 of the screw 10.

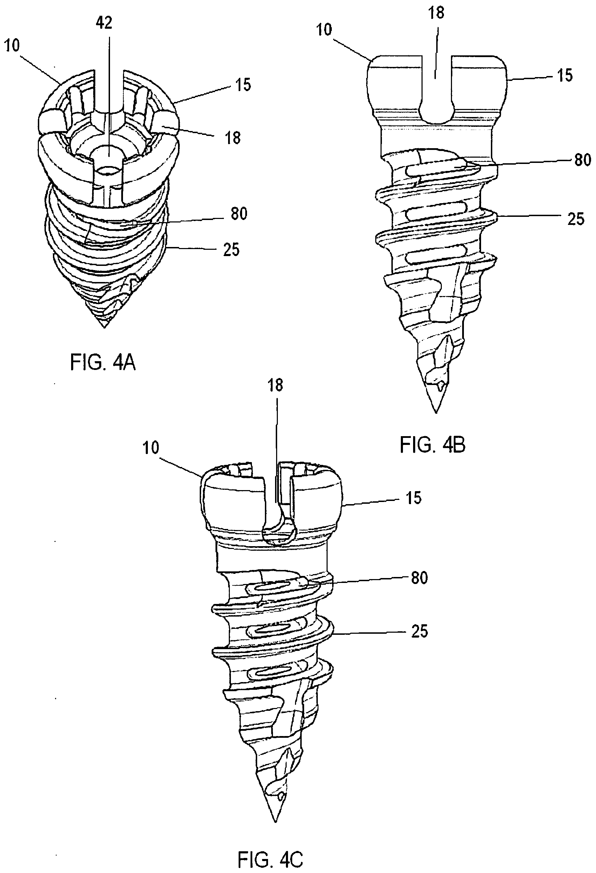

FIGS. 4A-4C illustrate an improved fenestrated screw having a plurality of angled, discontinuous fenestrations not falling within the scope of the claims. Like the screws discussed above, fenestrated screw 10 includes a head 15 and a shaft 25. However, the screw 10 includes a plurality of fenestrations 90. As shown in FIGS. 4B and 4C, the fenestrations 90 are slightly angled relative to a longitudinal mid-line of the screw 10. In addition, the fenestrations 90 are illustrated as substantially parallel to one another, although in other embodiments, the fenestrations 90 can be at non-parallel angles relative to one another. In the illustrated embodiment, there are three fenestrations 90; however, in other embodiments, there can be single, double, quadruple or more fenestrations. Advantageously, by having multiple fenestrations 90, the fenestrations 90 can distributed with ease around different portions of the screw, while maintaining the integrity of the strength of the screw. In some embodiments, one or more of the fenestrations 90 opens toward and is in fluidic contact with the inner hole 42 of the screw 10.

The fenestrated screws described above can be used in a variety of medical procedures, including surgery of the spine adjacent the lumbar, thoracic and even cervical vertebrae. While it has been difficult to include screws with fenestrations in cervical vertebrae, as such screws are small in size and may have reduced strength due to fenestrations, it has been found that performing a process on the screw material, such as shot-peening or cold-working, can enhance the fatigue life and postpone the development of surface cracks that may be found. Accordingly, in some embodiments, the material of the screws described above have been shot-peened or cold-worked in order to produce small, yet strong, screws for use in the cervical region of the vertebrae. In addition, by shot-peening or cold-working the material of the screws, this can increase the strength of the screws, thereby enhancing the pull out strength of a screw without negatively impacting the strength of the overall screw.

While the invention herein disclosed has been described by means of specific embodiments and applications thereof, numerous modifications and variations can be made thereto by those skilled in the art without departing from the scope of the invention.

## Claims

1. A bone screw (10) comprising:
- a head portion (15) comprising one or more longitudinal slits (18);
- a shaft portion (25) connected to the head portion (15), the shaft portion (25) comprising an outer surface having a plurality of threads and a tapered distal end, the shaft portion (25) further comprising a fenestration (60) extending along a majority of the length of the threaded shaft portion and having a first section (62) and a second section (64) extending along the outer surface, wherein the first section (62) extends along a first direction and the second section (64) extends along a second direction angled relative to the first direction, wherein the first section (62) begins at a proximal end of bone screw (10) near the head portion (15) and transitions into the second section (64) via an angled corner, wherein the first section (62) comprises a straight portion that extends along a longitudinal length of the shaft portion (25) which transitions via the angled corner into the second section (64),
wherein the one or more longitudinal slits (18) are configured to allow for compressibility of the head portion (15); and
- an inner opening (42) extending from the head portion (15) to the shaft portion (25), wherein the inner opening (42) is in fluid contact with fenestration (60) to distribute material introduced in the inner opening (42) through fenestration (60).

2. The bone screw of claim 1, wherein the head portion (15) comprises four symmetrical slits (18).

3. The bone screw of claim 1, wherein the shaft portion comprises dual-diameter threading.

4. The bone screw of claim 1, wherein the first section (62) of the fenestration (60) is substantially linear and extends substantially on a midline of the bone screw (10).

5. The bone screw of claim 1, the inner opening (42) is_a cannulated opening (42) that extends through the head portion (15) and the shaft portion (25).

6. The bone screw of claim 1, wherein at least a portion of the bone screw is shot-peened.

7. The bone screw of claim 1, wherein at least a portion of the bone screw is cold-worked.

8. The bone screw of claim 1, wherein it is configured for use in a cervical region of a spine.

9. The bone screw of claim 1, wherein the fenestration (60) extends along a majority of the length of the screw.

10. The bone screw of claim 1, wherein one of the one or more longitudinal slits (18) is generally aligned with the first section (62) of the fenestration (60).

11. The bone screw of claim 1, wherein the longitudinal slits (18) extends from an outer surface to an inner surface of the head portion (15).

12. The bone screw of claim 1, wherein the first direction is parallel to a central longitudinal axis and the first section (62) extends from a first position proximate to the head portion (15) to a second position a distance along the shaft portion and the second section (64) extends along a second direction angled relative to the first direction.

## Patentansprüche

1. Knochenschraube (10), umfassend:
- einen Kopfbereich (15) mit einem oder mehreren Längsschlitzen (18);
- einen Schaftbereich (25), der mit dem Kopfbereich (15) verbunden ist, wobei der Schaftbereich (25) eine äußere Fläche mit einer Mehrzahl von Gewinden und ein konisches distales Ende aufweist, wobei der Schaftbereich (25) ferner eine Fenestration (60) aufweist, die sich entlang eines Großteils der Länge des Schaftgewindebereichs erstreckt, und einen ersten Abschnitt (62) und einen zweiten Abschnitt (64) aufweist, die sich entlang der äußeren Fläche erstrecken, wobei sich der erste Abschnitt (62) entlang einer ersten Richtung und der zweite Abschnitt (64) entlang einer zweiten Richtung erstreckt, die relativ zur ersten Richtung abgeschrägt ist, wobei der erste Abschnitt (62) an einem proximalen Ende der Knochenschraube (10) in der Nähe des Kopfbereichs (15) beginnt, und sich in den zweiten Abschnitt (64) über eine abgeschrägte Ecke verändert, wobei der erste Abschnitt (62) einen geraden Bereich aufweist, der sich entlang einer Länge in Längsrichtung des Schaftbereichs (25) erstreckt, der sich über die abgeschrägte Ecke in den zweiten Abschnitt (64) verändert,
- wobei der eine oder mehrere Längsschlitze (18) eingerichtet sind, um eine Kompressibilität des Kopfbereichs (15) zu ermöglichen; und
- eine innere Öffnung (42), die sich vom Kopfbereich (15) zum Schaftbereich (25) erstreckt, wobei die innere Öffnung (42) in Fluidkontakt mit der Fenestration (60) ist, um Material zu verteilen, das in die innere Öffnung (42) durch die Fenestration (60) eingeführt wird.

2. Knochenschraube gemäß Anspruch 1, wobei der Kopfbereich (15) vier symmetrische Schlitze (18) aufweist.

3. Knochenschraube gemäß Anspruch 1, wobei der Schaftbereich ein Gewinde mit doppeltem Durchmesser aufweist.

4. Knochenschraube gemäß Anspruch 1, wobei der erste Abschnitt (62) der Fenestration (60) im Wesentlichen linear ist und sich im Wesentlichen auf einer Mittellinie der Knochenschraube (10) erstreckt.

5. Knochenschraube gemäß Anspruch 1, wobei die innere Öffnung (42) eine kanülierte Öffnung (42) ist, die sich durch den Kopfbereich (15) und den Schaftbereich (25) erstreckt.

6. Knochenschraube gemäß Anspruch 1, wobei zumindest ein Bereich der Knochenschraube kugelgestrahlt ist.

7. Knochenschraube gemäß Anspruch 1, wobei zumindest ein Bereich der Knochenschraube kalt verformt ist.

8. Knochenschraube gemäß Anspruch 1, wobei sie zum Verwenden in einem Halswirbelbereich einer Wirbelsäule eingerichtet ist.

9. Knochenschraube gemäß Anspruch 1, wobei sich die Fenestration (60) entlang eines Großteils der Länge der Schraube erstreckt.

10. Knochenschraube gemäß Anspruch 1, wobei einer von dem einen oder mehreren Längsschlitzen (18) im Wesentlichen mit dem ersten Abschnitt (62) der Fenestration (60) ausgerichtet ist.

11. Knochenschraube gemäß Anspruch 1, wobei sich der Längsschlitz (18) von einer äußeren Fläche zu einer inneren Fläche des Kopfbereichs (15) erstreckt.

12. Knochenschraube gemäß Anspruch 1, wobei die erste Richtung parallel zu einer mittleren Längsachse ist und sich der erste Abschnitt (62) von einer ersten Position, die nahe zum Kopfbereich (15) ist, zu einer zweiten Position mit einem Abstand entlang des Schaftbereichs erstreckt und sich der zweite Abschnitt (64) entlang einer zweiten Richtung erstreckt, die relativ zur ersten Richtung abgeschrägt ist.

## Revendications

1. Vis à os (10) comprenant :
- une partie tête (15) comprenant une ou plusieurs fentes longitudinales (18) ;
- une partie tige (25) reliée à la partie tête (15), la partie tige (25) comprenant une surface extérieure ayant une pluralité de filets et une extrémité distale effilée, la partie tige (25) comprenant en outre une fenêtre (60) s'étendant le long d'une majorité de la longueur de la partie tige filetée et ayant une première section (62) et une seconde section (64) s'étendant le long de la surface extérieure, dans laquelle la première section (62) s'étend le long d'une première direction et la seconde section (64) s'étend le long d'une seconde direction inclinée par rapport à la première direction, dans laquelle la première section (62) commence au niveau d'une extrémité proximale de la vis à os (10) près de la partie tête (15) et passe dans la seconde section (64) par le biais d'un coin incliné, dans laquelle la première section (62) comprend une partie droite qui s'étend le long d'une longueur longitudinale de la partie tige (25) qui passe par le biais du coin incliné dans la seconde section (64), dans laquelle la ou les fentes longitudinales (18) sont configurées pour permettre la compressibilité de la partie tête (15) ; et
- une ouverture intérieure (42) s'étendant depuis la partie tête (15) jusqu'à la partie tige (25), dans laquelle l'ouverture intérieure (42) est en contact fluidique avec la fenêtre (60) pour distribuer la matière introduite dans l'ouverture intérieure (42) par l'intermédiaire de la fenêtre (60).

2. Vis à os selon la revendication 1, dans laquelle la partie tête (15) comprend quatre fentes symétriques (18).

3. Vis à os selon la revendication 1, dans laquelle la partie tige comprend un filetage à deux diamètres.

4. Vis à os selon la revendication 1, dans laquelle la première section (62) de la fenêtre (60) est sensiblement linéaire et s'étend sensiblement sur une ligne médiane de la vis à os (10).

5. Vis à os selon la revendication 1, l'ouverture intérieure (42) est une ouverture canulée (42) qui s'étend à travers la partie tête (15) et la partie tige (25).

6. Vis à os selon la revendication 1, dans laquelle au moins une partie de la vis à os est grenaillée.

7. Vis à os selon la revendication 1, dans laquelle au moins une partie de la vis à os est travaillée à froid.

8. Vis à os selon la revendication 1, dans laquelle elle est configurée pour être utilisée dans une région cervicale d'une colonne vertébrale.

9. Vis à os selon la revendication 1, dans laquelle la fenêtre (60) s'étend le long d'une majorité de la longueur de la vis.

10. Vis à os selon la revendication 1, dans laquelle l'une des une ou plusieurs fentes longitudinales (18) est généralement alignée sur la première section (62) de la fenêtre (60).

11. Vis à os selon la revendication 1, dans laquelle les fentes longitudinales (18) s'étendent depuis une surface extérieure jusqu'à une surface intérieure de la partie tête (15).

12. Vis à os selon la revendication 1, dans laquelle la première direction est parallèle à un axe longitudinal central et la première section (62) s'étend depuis une première position à proximité de la partie tête (15) jusqu'à une seconde position sur une distance le long de la partie tige et la seconde section (64) s'étend le long d'une seconde direction inclinée par rapport à la première direction.
